# EUROPEAN PATENT APPLICATION

(11) **EP 2 053 058 A1**
(43) Date of publication of application: **29.04.2009**
(21) Application number: 07291290.0
(22) Date of filing: 24.10.2007
(51) Int. Cl.: C07K 14/25

(54) **Attenuation of bacterial virulence**

(71) Applicant: Imperial Innovations Limited, Imperial College London SW7 2AZ (GB)
(72) Inventor: The designation of the inventor has not yet been filed
(74) Representative: Senior, Janet

(57) **Abstract**

This application relates to compounds capable of inhibiting FNR transcription factor activity in a bacterial pathogen, methods of treating or preventing a pathogenic infection caused by a pathogenic bacteria by administering to a human, animal or plant said compounds, and methods of screening for compounds capable of inhibiting FNR transcription factor activity.

## Description

### TECHNICAL FIELD

The present invention relates to the attenuation of bacterial virulence. In particular, the present invention relates to the attenuation of virulence of bacterial pathogens that express a type three secretory system by inhibiting fnr transcription factor activity.

### BACKGROUND OF THE INVENTION

Pathogenic bacteria have a remarkable capacity to sense and respond to specific environmental cues. This capacity enables pathogens to co-ordinate the regulation of virulence factor expression in specific niches in the host. For example, enteric pathogens must survive in the largely anaerobic environment of the large intestine, and many share mechanisms both for sensing of lack of oxygen, and for promoting virulence.

*Shigella* spp. are the leading cause of bacillary dysentery, and a serious public health problem in developing countries. There are approximately 150 million episodes of shigellosis each year which contribute to failure to thrive and malnutrition, and result in around 1 million deaths. Shigellosis is disease of poverty, with the overwhelming burden of infection falling on children living in less wealthy countries with poor access to clean water and sanitation.

Shigellosis is characterised by intense inflammation in the lower GI tract mucosa, with recruitment of polymorphonuclear leukocytes (PMNs) to the site of infection, and loss of the intestinal epithelial cell (IEC) barrier with destruction of intestinal villi. The pathological changes seen during shigellosis are entirely dependent on the ability of *Shigella* to invade epithelial cells. Non-invasive shigellae are avirulent, and the characteristic inflammatory response to infection is initiated only when bacteria reach the intracellular compartment.

To invade into non-phagocytic cells, *Shigella* must express a type three secretion system (TTSS), that allows secretion of effector molecules directly into host cells from the bacterial cytoplasm. TTSSs are essential virulence factors for many bacteria including *Salmonella* spp., *Yersinia* spp., pathotypes of *Escherichia coli,* and *Chlamydia* spp.. Some *Shigella* effectors trigger extensive rearrangement of cytoskeleton (resulting in bacterial entry), while others modify the inflammatory response. All components necessary for expression of the TTSS and most effectors are encoded in a 32 kb *mxi-spa* pathogenicity island on the large virulence plasmid. Following cell entry, the bacterium rapidly escapes the nascent phagosome and enters the cytoplasm of thermaded cells in which it undergoes actin-based motility and cell to cell spread.

The environment of the GI tract plays a major role in regulating the virulence of *Shigella* and other pathogens. Genes in the *mxi-spa* locus are repressed at low temperatures, while pH and osmotic pressure also modulate virulence factors. However the effect of the low oxygen tensions found in the large intestine, the primary site of shigellosis, on *Shigella* has not been characterised to date. Moreover, the local environment for bacteria during the disease process is poorly understood. How the local environment changes in relation to specific sites in the GI tract, in the mucous layer, within crypts, and following invasion of epithelial cells and the subsequent changes to the mucosa is not certain.

### SUMMARY OF THE INVENTION

According to a first aspect, the invention provides a compound capable of inhibiting FNR transcription factor activity in a bacterial pathogen.

According to a second aspect, the invention provides a method of treating or preventing a pathogenic infection caused by a pathogenic bacteria by administering to a human, animal or plant a compound as according to the first aspect.

According to a third aspect, the invention provides a compound as defined for use in the treatment of a disease caused by a bacterial pathogen.

According to a fourth aspect, the invention provides a method of screening for compounds capable of inhibiting FNR transcription factor activity comprising the following steps:
i) contacting a candidate compound with a bacteria expressing FNR and comprising a genetic construct comprising an FNR-dependent promoter operably linked to a reporter gene;
ii) detecting inhibition of expression of the reporter gene.

### DESCRIPTION OF THE DRAWINGS

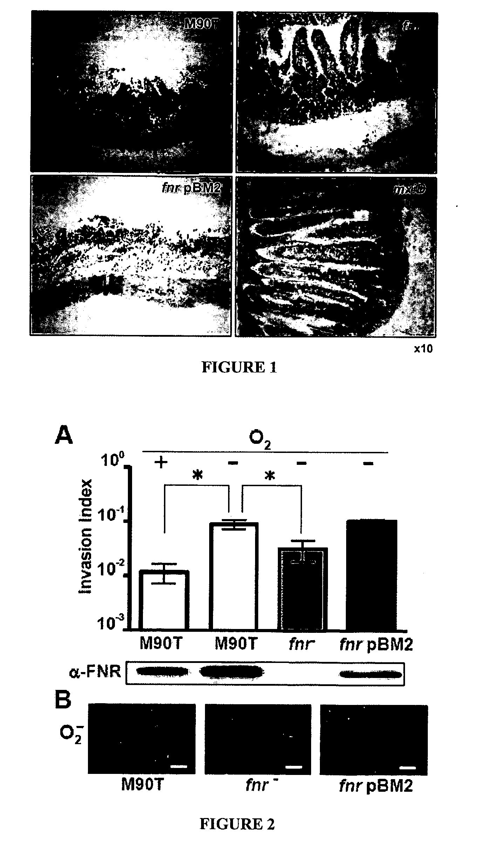

Figure 1 shows histopathological changes following challenge of rabbit ileal loops with the *fnr* mutant, wild-type (M90T), the complemented strain (*fnr* pBM2) and a non-invasive strain (*mxiD*)*.*
Figure 2 shows the influence of anaerobiasis and FNR on *Shigella* invasion. (A) The Invasion Index of Hela cells infected with wild-type *S*. *flexneri, fnr* mutant and complemented strains at an MOI 100; asterisks indicate p<0.05 (Student's T test). "α-FNR" represents the Western analysis of proteins from strains probed with anti-FNR antibodies. (B) Immunofluorescence analysis of bacterial invasion under anaerobic conditions, stained with anti-LPS pAbs (white dots) and phalloidin-TXR (to stain actin, shown grey). Bars, 10 µM.

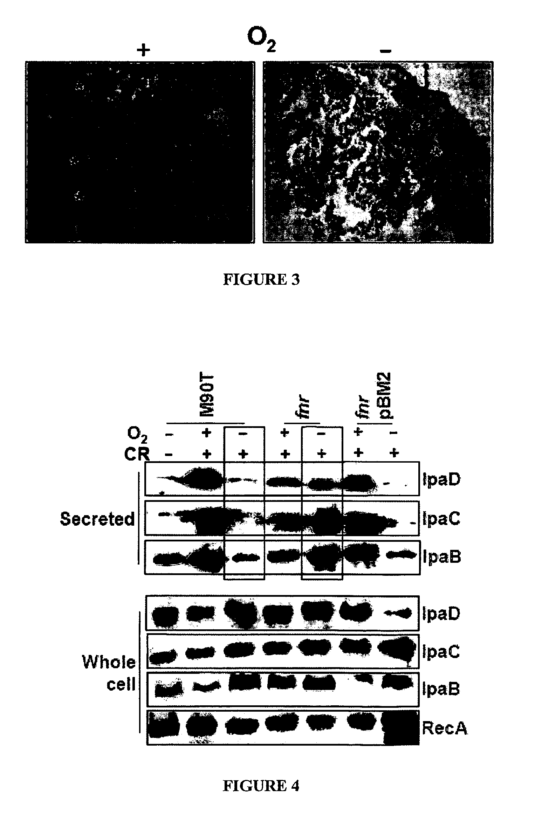

Figure 3 shows a histological analysis of rabbit ileal loops infected with the wild-type *Shigella* strain (M90T) grown aerobically or anaerobically (indicated with a + or -, respectively). Bacteria were detected with an anti-LPS antibody, and sections counterstained with Evans blue. Lumenal crypts are shown by asterisks.
Figure 4 shows the secretion of Invasion protein antigens (Ipas) through the Type Three Secretion System. Western analysis of secreted (A) and proteins from whole cells (B) using antibodies as indicated. *fnr* pBM2 is the complemented mutant. Secretion was induced by Congo Red (CR) where indicated. Vertical boxes highlight the FNR-dependent lack of Ipa secretion in the absence of oxygen.

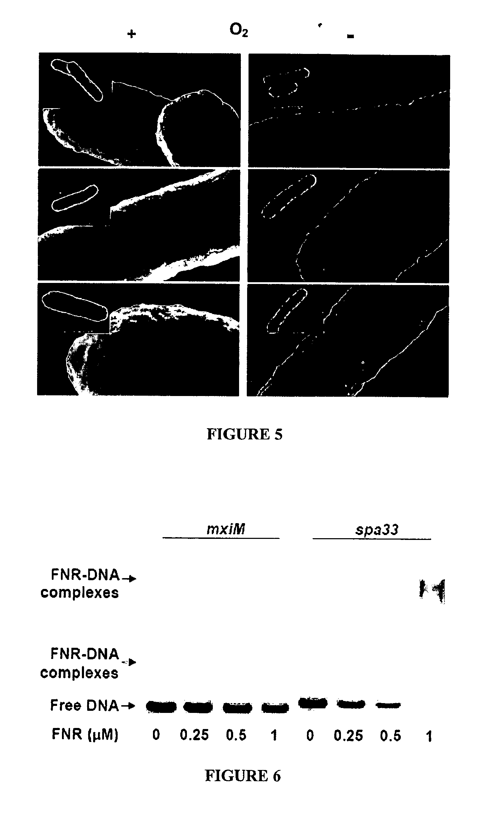

Figure 5 shows an examination by scanning EM of *S. flexneri* grown under aerobic (+) or anaerobic (-) conditions. Arrows indicate the location of secretons at the bacterial surface.
Figure 6 shows the binding of purified FNR D¹⁵⁴A to DNA fragments of regions upstream of *spa33* and *mxiM* genes. Electrophoretic Mobility Shift Assays with end-labelled *Eco*RI*-Hin*DIII DNA fragments are illustrated. DNA fragments were incubated with 0, 0.25, 0.5 or 1.0 µM FNR as indicated.

### DEFINITIONS

FNR means any bacterial protein having a biological function corresponding to FNR in *Shigella.* Preferably said protein has at least 40%, more preferably at least 45%, more preferably at least 50%, more preferably at least 55%, more preferably at least 60%, more preferably at least 65%, more preferably at least 70%, more preferably at least 75%, more preferably at least 80%, more preferably at least 85%, more preferably at least 90% homology at the amino acid level with *Shigella* FNR.

### DETAILED DESCRIPTION OF THE INVENTION

FNR is a DNA binding protein originally named fumarate nitrate reduction (because it was erroneously thought to have nitrate reductase activity).

The FNR molecule includes a Fe-S cluster which is able to change its oxidation state in response to changes in oxygenation of the environment. It is thought that this change in oxidation state leads to a conformational change such that at low oxygen tensions the FNR molecule has a conformation which permits dimerization and DNA binding. At higher oxygen tensions the FNR molecule is thought to adopt a conformation which does not permit dimerization nor DNA binding.

The present invention is based on the demonstration that the FNR protein mediates virulence in Shigella (see Examples 1 and 2) and that non functional fnr mutants of *Shigella* which are unable to encode FNR with the ability to dimerize and act as a transcription factor show reduced pathogenicity (see Example 3).

It is further demonstrated that FNR mediates pathogenicity of *Shigella* by modulating transcription of a number of virulence genes (see Example 6) including those of the TTSS (see Example 4) which is important for *Shigella* invasiveness (see Example 5).

FNR is found in many bacterial pathogens (see Example 7).

Accordingly, according to a first aspect of the invention the present invention provides a compound for the inhibition of FNR transcription factor activity in a bacterial pathogen.

Said compound may inhibit FNR transcription factor activity by inhibiting dimerization of active FNR monomers. For example, said compound may be an antibody (or epitope binding fragment or derivative thereof) capable of binding to an FNR monomer so as to prevent dimerization, or so as to inhibit the conformational changes in response to low oxygen tension. Alternatively, said compounds may inhibit FNR transcription factor activity by disrupting binding of FNR dimers to target DNA sequences.

According to certain embodiments said compound is an antisense nucleic acid molecule capable of hybridizing to the following DNA consensus binding sequence under conditions found *in vivo:*
TTGAT (N₄) ATCAA where N is any residue.

According to other embodiments said compound is an antisense molecule capable of reducing FNR expression.

Preferably said compound inhibits the transcription of genes of the TTSS.

Preferably said compound inhibits cellular invasion.

Said bacterial pathogen is preferably a human pathogen, although in certain embodiments said bacterial pathogen may be a pathogen of non-human animals or of plants.

Bacteria known to possess both fnr and genes involved in TTSS biosynthesis include *Burkholderia pseudomallei, Dechloromonas aromatica, Erwinia carotovora atroseptica, Escherichia coli, Mesorhizobium loti, Pseudomonas fluorescens, Ralstonia eutropha, Ralstonia solanacearum, Salmonella enterica Choleraesuis, Salmonella enterica Paratyphi, Salmonella enterica serovar Typhi, Vibrio parahaemolyticus, Xanthomonas campestris, Xanthomonas oryzae, Yersinia pestis,* and *Yersinia pseudotuberculosis*

Preferably said bacterial pathogen is a gram negative pathogen, for example, *Pseudomonas, Klebsiella, Salmonella, E.coli* and *Shigella,* most preferably *Shigella.*

Alternatively said bacterial pathogen is a gram positive pathogen, for example, *Bacillus,* especially *B anthracis* or *Clostridium,* especially *C.difficile.*

According to a second embodiment, the present invention provides a method of treating or preventing a pathogenic infection caused by a pathogenic bacteria according to the first aspect of the invention by administering to a human, animal or plant a compound as defined according to the first aspect of the invention.

According to a third embodiment, the present invention provides a compound according to the first embodiment for use in treating a human or animal disease caused by a bacterial pathogen as defined according to the first aspect of the invention.

Preferably said disease is bacillary dysentery caused by *Shigella.*

According to a fourth embodiment, the present invention provides a method of screening for compounds capable of inhibiting FNR transcription factor activity comprising the following steps:
i) contacting a candidate compound with a bacteria expressing FNR and comprising a genetic construct comprising an FNR-dependent promoter operably linked to a reporter gene;
ii) detecting inhibition of expression of the reporter gene.

Said method may be used for screening for compounds of use as anti-microbial agents, in particular agents active against the bacterial pathogens mentioned herein, for example use for screening for compounds active against *Shigella* invasion for use in the treatment for dysentery.

Preferably said method can be applied to high throughput screening.

Said method may be carried out under anaerobic conditions but for convenience it is preferred that said bacteria comprises a constitutively active version of FNR (for example that disclosed in reference 4) which is not dependent on lack of oxygen to be able to bind to DNA and stimulate gene transcription. Such a method may therefore be carried out under ambient conditions.

Said method may be carried out using any bacteria containing FNR. However, for convenience and safety it is preferred to use a bacteria of low potential pathogenicity, for example, a disabled *E*.*coli* bacteria which may be safely grown with non-onerous safety precautions. If *E.coli* is used, it would be possible to use the endogenous *E.coli* FNR. Alternatively, the *E.coli* bacteria may be engineered to express an exogenous FNR protein, for example an FNR protein from other bacterial pathogens for example from *Shigella.*

Whilst the method described above is particularly suited to high throughput screening, the invention also encompasses methods of screening for antibacterial compounds by use of the rabbit ileal loop model described in Example 3.

According to a fifth embodiment, the invention provides a compound obtainable by a method of the fourth embodiment.

Further aspects of the invention are described below:

### Antisense Therapy

FNR function may be inhibited by use of *fnr* antisense nucleic acids. The present invention provides the therapeutic or prophylactic use of nucleic acids of at least six nucleotides and are preferably oligonucleotides (ranging from 6 to about 200 oligonucleotides), that are antisense to a gene or cDNA encoding an FNR protein, or portions thereof. An *fnr* "antisense" nucleic acid as used herein refers to a nucleic acid capable of hybridizing to a portion of an *fnr* nucleic acid (preferably mRNA) by virtue of some sequence complementarity. The antisense nucleic acid may be complementary to a coding and/or noncoding region of a *fnr* mRNA. In specific aspects, the oligonucleotide is at least 10 nucleotides, at least 15 nucleotides, at least 100 nucleotides, or at least 200 nucleotides.

The oligonucleotides can be DNA or RNA or chimeric mixtures or derivatives or modified versions thereof, single-stranded or double-stranded. The oligonucleotide can be modified at any position (examples of such modifications can be found in: Bailey, Ullmann's Encyclopedia of Industrial Chemistry (1998), 6th ed. Wiley and Sons). Such antisense nucleic acids have utility as Therapeutics that inhibit fnr function or activity. These anti-sense-oligonucleotides may act by binding to the polypeptides coding for fnr or mRNAs corresponding thereto and thereby inhibiting the transcription or translation thereof, promoting the degradation of the mRNAs, and/or inhibiting the expression of the proteins encoded by the nucleotides, and finally inhibiting the function of the proteins.

The nucleic acids that inhibit one or more gene products also include small interfering RNAs (siRNA) comprising a combination of a sense strand nucleic acid and an antisense strand nucleic acid of a nucleotide sequence coding for fnr. The term "siRNA" refers to a double stranded RNA molecule which prevents translation of a target mRNA. The siRNA is constructed such that a single transcript has both the sense and complementary antisense sequences from the target gene, e.g., a hairpin.

The nucleotide sequence of siRNAs may be designed using a siRNA design computer program available from the Ambion website

(http://www.ambion.com/techlib/misc/siRNA_finder.html). Nucleotide sequences for the siRNA are selected by the computer program based on the following protocol:

### Selection of siRNA Target Sites:

1. Beginning with the AUG start codon of transcript, scan downstream for AA dinucleotide sequences. Record the occurrence of each AA and the 3' adjacent 19 nucleotides as potential siRNA target sites. Tusehi, et al. recommend not to design siRNA against the 5' and 3' untranslated regions (UTRs) and regions near the start codon (within 75 bases) as these may be richer in regulatory protein binding sites, and thus the complex of endonuclease and siRNAs that were designed against these regions may interfere with the binding of UTR-binding proteins and/or translation initiation complexes.
2. Compare the potential target sites to the human genome database and eliminate from consideration any target sequences with significant homology to other coding sequences. The homology search can be performed using BLAST, which can be found on the NCBI server at: www.ncbi.nlm.nih.gov/BLAST/
3. Select qualifying target sequences for synthesis. On the website of Ambion, several preferable target sequences can be selected along the length of the gene for evaluation.

The *fnr* antisense nucleic acids can be directly administered to a bacterial cell, or can be produced intracellularly by transcription of exogenous, introduced sequences.
Alternatively, *fnr* antisense nucleic acids are produced intracellularly by transcription from an exogenous sequence. For example, a vector, for example a bacteriophage, can be introduced *in vivo* such that it is taken up by a bacterial cell, within which cell the vector or a portion thereof is transcribed, producing an antisense nucleic acid (RNA) of the invention. Such a vector can remain episomal or become chromosomally integrated, as long as it can be transcribed to produce the desired antisense RNA. Such vectors can be constructed by recombinant DNA technology methods standard in the art.

The antisense nucleic acids of the invention comprise a sequence complementary to at least a portion of an RNA transcript of an *fnr* gene. However, absolute complementarity, although preferred, is not required.

Pharmaceutical compositions of the invention, comprising an effective amount of an *fnr* antisense nucleic acid in a pharmaceutically acceptable carrier can be administered to a patient having a disease or disorder caused by a bacterial pathogen.

The amount of *fnr* antisense nucleic acid that will be effective in the treatment of a particular disorder or condition will depend on the nature of the disorder or condition, and can be determined by standard clinical techniques. Where possible, it is desirable to determine the antisense cytotoxicity *in vitro,* and then in useful animal model systems prior to testing and use in humans.

In a specific embodiment, pharmaceutical compositions comprising *fnr* antisense nucleic acids are administered via liposomes, microparticles, or microcapsules. In various embodiments of the invention, it may be useful to use such compositions to achieve sustained release of the *fnr* antisense nucleic acids. In a specific embodiment, it may be desirable to utilize liposomes targeted via antibodies to specific identifiable central nervous system cell types (Leonetti et al., 1990, Proc. Natl. Acad. Sci. U.S.A. 87: 2448-2451; Renneisen et al., 1990, J. Biol. Chem. 265: 16337-16342).

### Generation of Antibodies to FNR Proteins

FNR proteins, including functional derivatives and fragments thereof may be used as an immunogen to generate monoclonal or polyclonal antibodies and antibody fragments or derivatives (*e.g.*, chimeric, single chain, Fab fragment, etc.). For example, antibodies to a particular domain of an FNR protein may be desired. In a specific embodiment, fragments of an FNR protein identified as hydrophilic are used as immunogens for antibody production using art-known methods.

Antibody and antibody fragments for use in the present invention can be generated by a number of known artificial and natural processes as discussed below.

In one embodiment the antibody fragment may be Fab, Fab', F(ab')2, Fv or single chain Fv (scFv), in which Fv fragments from H and L chains are ligated by an appropriate linker (Huston et al, Proc. Natl. Acad. Sci. USA, 85:5879-83, 1988). More specifically, an antibody fragment may be generated by treating an antibody with an enzyme, such as papain or pepsin. Alternatively, a gene encoding the antibody fragment may be constructed, inserted into an expression vector, and expressed in an appropriate host cell (see, for example, Co et al, J. Immunol, 152:2968-76, 1994; Better and Horwitz, Methods Enzymol., 178:476-96, 1989; Pluckthun and Skerra, Methods Enzymol, 178:497-515, 1989; Lanioyi, Methods Enzymol, 121 :652-63, 1986; Rousseaux et a!., Methods Enzymol., 121 :663-9, 1986; Bird and Walker, Trends Biotechnol, 9:132-7, 1991).

An antibody may be modified by conjugation with a variety of molecules, such as polyethylene glycol (PEG). The modified antibody can be obtained by chemically modifying an antibody. These modification methods are conventional in the field.

Alternatively, an antibody may be obtained as a chimeric antibody, between a variable region derived from non-human antibody and the constant region derived from human antibody, or as a humanized antibody, comprising the complementarity determining region (CDR) derived from non-human antibody, the frame work region (FR) derived from human antibody, and the constant region. Such antibodies can be prepared using known art methods.

In brief, methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include a FNR protein, a functional derivative or fragments thereof, or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL TDM adjuvant (moriophosphoryl Lipid A, synthetic trehalose dicorynomycolate). The immunization protocol may be selected by one skilled in the art without undue experimentation.

Intracellular antibodies are generally single chain antibodies herein they will comprise single chain antibodies which specifically bind a FNR protein. They may be used in gene therapy by incorporating the sequence encoding the antibody into a recombinant vector and administered to cells expressing a FNR protein to bind and inhibit its function. Methods for producing these antibodies are known in the art. (see for example Tanaka et al, Nucleic Acids Research 31 (5):e23 (2003)

Monoclonal antibodies may be prepared using hybridoma methods, such as those described by Kohler and Milstein {Nature, 256:495, 1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro.

The immunizing agent will typically include the FNR protein, a functional derivative or fragment thereof, or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (see, e.g., Goding, Monoclonal Antibodies: Principles and Practice, Academic Press, pp. 59-103, 1986). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), that will prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [J. Immunol., 133:3001, 1984; Brodeur et al, Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against the FNR protein. Preferably, the binding specificity of monoclonal antibodies produced by the hybridoma cells is determined by immunoprecipitation or by an in vitro binding assay, such as radio linked immunoassay (RJA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220, 1980.

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI- 1640 medium. Alternatively, the hybridoma cells may be grown in vivo as ascites in a mammal.

The monoclonal antibodies secreted by the subclones may be isolated or purified from the culture medium or ascites fluid by conventional immunoglobulin purification procedures such as, for example, protein A-Sepharose, hydroxyapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies for use in the invention can be readily isolated and sequenced using conventional procedures (e.g., by using oligonucleotide probes that are capable of binding specifically to genes encoding the heavy and light chains of murine antibodies. The hybridoma cells serve as a preferred source of such DNA. Once isolated, the DNA may be placed into expression vectors, which are then transfected into host cells such as simian COS cell Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody for use in the invention, or can be substituted for the variable domains of one antigen-combining site of an antibody to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

In vitro methods are also suitable for preparing monovalent antibodies. Digestion of antibodies to produce fragments thereof, particularly, Fab fragments, can be accomplished using routine techniques known in the art.

The antibodies for use in the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues.

Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al, Nature, 321:522-525 (1986); Riechmann et al, Nature, 332:323- 329, 1988; and Presta, Curr. Op. Struct. Biol, A:593-596, 1992].

Methods for humanizing non-human antibodies are well known in the art. Generally, a humanized antibody has one or more amino acid residues introduced into it from a source which is non human. These non-human amino acid residues are often referred to as "import" residues, which are typically taken from an "import" variable domain. Humanization can be essentially performed following the method of Winter and coworkers [Jones et al, Nature, 321:522-525, 1986; Riechmann et al, Nature, 332:323-327, 1988; Verhoeyen et al, Science, 239:1534-1536, 1988], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol, 227:381 (1991); Marks et al, J. Mol Biol, 222:581 (1991)]. The techniques of Cole et al and Boerner et al are also available for the preparation of human monoclonal antibodies (Cole et al, Monoclonal Antibodies and Cancer Therapy Alan R. Liss, p. 77 (1985) and Boerner et al, J. Immunol, 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al, Bio/Technology, 10:779-783 (1992); Lonberg et al, Nature, 368 856-859 (1994); Morrison, Nature, 368:812-13 (1994); Fishwild et al, Nature Biotechnology, 14:845-5 1 (1996); Neuberger, Nature Biotechnology, 14:826 (1996); Lonberg and Huszar, Intern. Rev. Immunol, 13:65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

Bi-specific antibodies are monoclonal, preferably human or humanized, antibodies that have binding specificities for at least two different antigens. In the present case, one of the binding specificities is for the FNR protein, the other one is for any other antigen, and preferably for a cell-surface protein or receptor or receptor subunit.

Methods for making bi-specific antibodies are known in the art. Traditionally, the recombinant production of bi-specific antibodies is based on the co-expression of two immunoglobulin heavy chain light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture often different antibody molecules, of which only one has the correct bi- specific structure. The purification of the correct molecule is usually accomplished by affinity chromatography steps. Similar procedures are disclosed in PCT Intl. Pat. Appl. Publ. No. WO 93/08829, and in Traunecker et al, EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy- chain fusions and, if desired, the immunoglobulin light chain, are inserted into separate expression vectors, and are co-transfected into a suitable host organism. For further details of generating bi-specific antibodies see, for example, Suresh et al, Methods Enzymol, 121:210 (1986).

According to another approach described in PCT Intl. Pat. Appl. Publ. No. WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bi-specific antibodies can be prepared as full length antibodies or antibody fragments (e.g., F(ab')2 bi-specific antibodies). Techniques for generating bi-specific antibodies from antibody fragments have been described in the literature. For example, bi-specific antibodies can be prepared using chemical linkage. Brennan et al, Science, 229:81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bi-specific antibody. The bi- specific antibodies produced can be used as agents for the selective immobilization of enzymes.

Fab' fragments may be directly recovered from E. coli and chemically coupled to form bi-specific antibodies. Shalaby et al, (J. Exp. Med., 175:217-225, 1992) describe the production of a fully humanized bi-specific antibody F(ab') molecule. Each Fab' fragment was separately secreted from E. coli and subjected to directed chemical coupling in vitro to form the bi-specific antibody. The bi-specific antibody thus formed was able to bind to cells overexpressing the ErbB2 receptor and normal human T cells, as well as trigger the lytic activity of human cytotoxic lymphocytes against human breast tumor targets.

Various techniques for making and isolating bi-specific antibody fragments directly from recombinant cell culture have also been described. For example, bi-specific antibodies have been produced using leucine zippers. Kostelny et al, J. Immunol., 148(5): 1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al, (Proa Natl Acad. Sci. USA, 90:6444-6448, 1993) has provided an alternative mechanism for making bi-specific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bi- specific antibody fragments by the use of single-chain Fv (sFv) dimers has also been reported (See, e.g., Gruber et al, J. Immunol, 152:5368, 1994).

Antibodies with more than two valencies are contemplated. For example, trispecific antibodies can be prepared (see, e.g., Tutt et al, J. Immunol, 147:60, 1991).

Exemplary bi-specific antibodies may bind to two different epitopes on a given FNR protein herein. Alternatively, an anti-FNR arm may be combined with an arm which binds to a triggering molecule on a leukocyte such as a T-cell receptor molecule (e.g., CD2, CD3, CD28, or B7), or Fc receptors for IgG (FcγR), such as FcγRI (CD64), FcγRII (CD32) and FcγRII (CD 16) so as to focus cellular defense mechanisms to the cell expressing the particular FNR protein. Bi-specific antibodies may also be used to localize cytotoxic agents to cells which express a particular FNR homologue. These antibodies possess a FNR-binding arm and an arm which binds a cytotoxic agent or a radionuclide chelator, such as EOTUBE, DPTA, DOTA, or TETA. Another bi-specific antibody of interest binds the FNR protein and further binds tissue factor (TF).

Antiidiotypic antibodies can also be used in the therapies discussed herein, to induce an immune response to cells expressing a FNR protein. Production of these antibodies is also well known (see for example Wagner et al, Hybridoma 16:33-40 (1997)).

Heteroconjugate antibodies are also within the scope of the present invention. Heteroconjugate antibodies are composed of two covalently joined antibodies. Such antibodies have, for example, been proposed to target immune system cells to unwanted cells (see, e.g., U. S. Patent No. 4,676,980), and for treatment of HIV infection (PCT Intl. Pat. Appl. Publ. No. WO 91/00360; and PCT Intl. Pat. Appl. Publ. No. WO 92/200373). It is contemplated that the antibodies may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include immunothiolate and methyl-4-mercaptobutyriniidate and those disclosed, for example, in U. S. Patent No. 4,676,980.

It may be desirable to modify an antibody with respect to effector function, so as to enhance, e.g., the effectiveness of the antibody. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC) (See, e.g., Caron et al, J. Exp Med, 176:1191-1195, 1992 and Shopes, J. Immunol, 148:2918-2922, 1992). Homodimeric antibodies may also be prepared using heterobifunctional cross-linkers as described in Wolff et al. Cancer Research, 53:2560-2565, 1993. Alternatively, an antibody can be engineered that has dual Fc regions and may thereby have enhanced complement lysis and ADCC capabilities. See, e.g., Stevenson et al, Anti-Cancer Drug Design, 3:219-230, 1989.

### Candidate Therapeutics

Candidate therapeutics may come from any source of therapeutics known in the art. For example, these can be proteins, nucleic acids (including anti-sense nucleic acids), antibodies, peptides, organic molecules, etc. In some instances, compounds may be screened first in *in vitro* assays to determine their potential as anti-bacterial therapeutics.

For example, chemical libraries may be screened for useful therapeutics. Exemplary libraries are commercially available from several sources (ArQule, Tripos/PanLabs, ChemDesign, Pharmacopoeia). Many diversity libraries suitable for use are known in the art and can be used to provide compounds to be tested according to the present invention. Alternatively, libraries can be constructed using standard methods. Chemical (synthetic) libraries (Houghten et al., 1991, Nature 354:84-86; Lam et al., 1991, Nature 354:82-84; Medynski, 1994, Bio/Technology 12:709-710; Gallop et al., 1994, J. Medicinal Chemistry 37(9):1233-1251), recombinant expression libraries, or polysome-based libraries are exemplary types of libraries that can be used. Other examples include combinatorial libraries (Ohlmeyer et al., 1993, Proc. Natl. Acad. Sci. USA 90:10922-10926; Erb et al., 1994, Proc. Natl.Acad.Sci. USA 91:11422-11426; Houghten et al., 1992, Biotechniques 13:412; Jayawickreme et al., 1994, Proc. Natl. Acad. Sci. USA 91:1614-1618; Salmon et al., 1993, Proc. Natl. Acad. Sci. USA 90:11708-11712), organic diversity (e.g., nonpeptide) libraries (Bunin et al., 1994, Proc. Natl. Acad. Sci. USA 91:4708-4712) may be used. Libraries of non-peptides, e.g., peptide derivatives (for example, that contain one or more non-naturally occurring amino acids) can also be used. One example of these are peptoid libraries (Simon et al., 1992, Proc. Natl. Acad. Sci. USA 89:9367-9371). Peptoids are polymers of non-natural amino acids that have naturally occurring side chains attached not to the alpha carbon but to the backbone amino nitrogen. Since peptoids are not easily degraded by human digestive enzymes, they are advantageously more easily adaptable to drug use.

Variants of candidate proteins and nucleic acids may also be used as candidate therapeutics. As used herein, the term "variant" refers to polynucleotide or polypeptide sequences different from the specifically identified sequences, wherein one or more nucleotides or amino acid residues is deleted, substituted, or added. Variants may be naturally occurring allelic variants, or non-naturally occurring variants. Variants may be from the same or from other species and may encompass homologues, paralogues and orthologues. The term "variant" with reference to polynucleotides and polypeptides encompasses all forms of polynucleotides and polypeptides as defined herein.

### Polynucleotide variants

Variant polynucleotide sequences preferably exhibit at least 50%, more preferably at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% identity to a specified polynucleotide sequence. Identity is preferably found over a comparison window of at least 20 nucleotide positions, at least 50 nucleotide positions, at least 100 nucleotide positions, or over the entire length of the specified polynucleotide sequence.

Polynucleotide sequence identity can be determined in the following manner. The subject polynucleotide sequence is compared to a candidate polynucleotide sequence using BLASTN (from the BLAST suite of programs, version 2.2.15 [Oct 2006]) in b12seq (Tatiana A. Tatusova, Thomas L. Madden (1999), "Blast 2 sequences - a new tool for comparing protein and nucleotide sequences", FEMS Microbiol Lett. 174:247-250), which is publicly available from NCBI (ftp://flp.ncbi.nih.gov/blast/). The default parameters of b12seq are utilized except that filtering of low complexity parts should be turned off.

The identity of polynucleotide sequences may be examined using the following unix command line parameters:
b12seq - i nucleotideseq 1 -j nucleotideseq2 -F F -p blastn

The parameter -F F turns off filtering of low complexity sections. The parameter -p selects the appropriate algorithm for the pair of sequences. The b12seq program reports sequence identity as both the number and percentage of identical nucleotides in a line "Identities = ".

Polynucleotide sequence identity may also be calculated over the entire length of the overlap between a candidate and subject polynucleotide sequences using global sequence alignment programs (e.g. Needleman, S. B. and Wunsch, C. D. (1970) J. MoI. Biol. 48, 443-453). A full implementation of the Needleman-Wunsch global alignment algorithm is found in the needle program in the EMBOSS package (Rice,P. Longden,I. and Bleasby,A. EMBOSS: The European Molecular Biology Open Software Suite, Trends in Genetics June 2000, vol 16, No 6. pp.276-277) which can be obtained from http://www.hgmp.mrc.ac.uk/Software/EMBOSS/. The European Bioinformatics Institute server also provides the facility to perform EMBOS S-needle global alignments between two sequences on line at http:/www.ebi.ac.uk/emboss/align/.

Alternatively the GAP program may be used which computes an optimal global alignment of two sequences without penalizing terminal gaps. GAP is described in the following paper: Huang, X. (1994) On Global Sequence Alignment. Computer Applications in the Biosciences 10, 227-235.

Polynucleotide variants for use in the present invention also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available b12seq program from the BLAST suite of programs (version 2.2.15 [Oct 2006]) from NCBI fft:p://ftp.ncbi.nih.gov/blast/).

The similarity of polynucleotide sequences may be examined using the following unix command line parameters:
b12seq -i nucleotideseq1 -j nucleotideseq2 -F F -p tblastx

The parameter - F F turns off filtering of low complexity sections. The parameter - p selects the appropriate algorithm for the pair of sequences. This program finds regions of similarity between the sequences and for each such region reports an "E value" which is the expected number of times one could expect to see such a match by chance in a database of a fixed reference size containing random sequences. The size of this database is set by default in the bl2seq program. For small E values, much less than one, the E value is approximately the probability of such a random match.

Variant polynucleotide sequences preferably exhibit an E value of less than 1 x 10⁻¹⁰, more preferably less than 1 x 10⁻²⁰, less than 1 x 10⁻³⁰, less than 1 x 10⁻⁴⁰, less than 1 x 10⁻⁵⁰, less than 1 x 10⁻⁶⁰, less than 1 x 10⁻⁷⁰, less than 1 x 10⁻⁸⁰, less than 1 x 10⁻⁹⁰, less than 1 x 10⁻¹⁰⁰, less than 1 x 10⁻¹¹⁰, less than 1 x 10 ⁻¹²⁰, or less than 1 x 10 ⁻¹²³ when compared with any one of the specifically identified sequences.

Alternatively, variant polynucleotides for use in the present invention hybridize to a specified polynucleotide sequence, or complements thereof under stringent conditions.

The term "hybridize under stringent conditions", and grammatical equivalents thereof, refers to the ability of a polynucleotide molecule to hybridize to a target polynucleotide molecule (such as a target polynucleotide molecule immobilized on a DNA or RNA blot, such as a Southern blot or Northern blot) under defined conditions of temperature and salt concentration. The ability to hybridize under stringent hybridization conditions can be determined by initially hybridizing under less stringent conditions then increasing the stringency to the desired stringency.

With respect to polynucleotide molecules greater than about 100 bases in length, typical stringent hybridization conditions are no more than 25 to 30°C (for example, 10°C) below the melting temperature (Tm) of the native duplex (see generally, Sambrook et al, Eds, 1987, Molecular Cloning, A Laboratory Manual, 2nd Ed. Cold Spring Harbor Press; Ausubel et al, 1987, Current Protocols in Molecular Biology, Greene Publishing,). Tm for polynucleotide molecules greater than about 100 bases can be calculated by the formula Tm = 81. 5 + 0. 41 % (G + C-log (Na+). (Sambrook et al, Eds, 1987, Molecular Cloning, A Laboratory Manual, 2nd Ed. Cold Spring Harbor Press; Bolton and McCarthy, 1962, PNAS 84:1390). Typical stringent conditions for polynucleotide of greater than 100 bases in length would be hybridization conditions such as prewashing in a solution of 6X SSC, 0.2% SDS; hybridizing at 65°C, 6X SSC, 0.2% SDS overnight; followed by two washes of 30 minutes each in IX SSC, 0.1% SDS at 65°C and two washes of 30 minutes each in 0.2X SSC, 0.1% SDS at 65°C.

With respect to polynucleotide molecules having a length less than 100 bases, exemplary stringent hybridization conditions are 5 to 10°C below Tm. On average, the Tm of a polynucleotide molecule of length less than 100 bp is reduced by approximately (500/oligonucleotide length)°C.

With respect to the DNA mimics known as peptide nucleic acids (PNAs) (Nielsen et al, Science. 1991 Dec 6;254(5037): 1497-500) Tm values are higher than those for DNA-DNA or DNA-RNA hybrids, and can be calculated using the formula described in Giesen et al, Nucleic Acids Res. 1998 Nov 1;26(21):5004-6. Exemplary stringent hybridization conditions for a DNA- PNA hybrid having a length less than 100 bases are 5 to 10°C below the Tm.

Variant polynucleotides for use in the present invention also encompasses polynucleotides that differ from the sequences disclosed herein but that, as a consequence of the degeneracy of the genetic code, encode a polypeptide having similar activity to a polypeptide encoded by a specified polynucleotide. A sequence alteration that does not change the amino acid sequence of the polypeptide is a "silent variation". Except for ATG (methionine) and TGG (tryptophan), other codons for the same amino acid may be changed by art recognized techniques, e.g., to optimize codon expression in a particular host organism.

Polynucleotide sequence alterations resulting in conservative substitutions of one or several amino acids in the encoded polypeptide sequence without significantly altering its biological activity are also included in the invention. A skilled artisan will be aware of methods for making phenotypically silent amino acid substitutions (see, e.g., Bowie et al, 1990, Science 247, 1306).

Variant polynucleotides due to silent variations and conservative substitutions in the encoded polypeptide sequence may be determined using the publicly available b12seq program from the BLAST suite of programs (version 2.2.15 [Oct 2006]) from NCBI (ftp://ftp.ncbi.nih. gov/blast/) via the tblastx algorithm as previously described.

### Polypeptide Variants

The term "variant" with reference to polypeptides encompasses naturally occurring, recombinantly and synthetically produced polypeptides. Variant polypeptide sequences preferably exhibit at least 50%, more preferably at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71 %, at least 72%, at least 73%, at least 74%, at least 75%, at least 76%, at least %, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% identity to a specified sequence. Identity is preferably found over a comparison window of at least 20 amino acid positions, at least 50 amino acid positions, at least 100 amino acid positions, or over the entire length of a polypeptide.

Polypeptide sequence identity can be determined in the following manner. The subject polypeptide sequence is compared to a candidate polypeptide sequence using BLASTP (from the BLAST suite of programs, version 2.2.15 [Oct 2006]) in b12seq, which is publicly available from NCBI (ftp://ftp.ncbi.nih.gov/blast/). The default parameters of b12seq are utilized except that filtering of low complexity regions should be turned off.

Polypeptide sequence identity may also be calculated over the entire length of the overlap between a candidate and subject polynucleotide sequences using global sequence alignment programs. EMBOSS-needle (available at http://www.ebi.acuk/emboss/align/) and GAP (Huang, X. (1994) On Global Sequence Alignment. Computer Applications in the Biosciences 10, 227-235) as discussed above are also suitable global sequence alignment programs for calculating polypeptide sequence identity.

Polypeptide variants for use in the present invention also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available b12seq program from the BLAST suite of programs (version 2.2.15 [Oct 2006]) from NCBI (ftp://ftp.ncbi.nih.gov/blast/). The similarity of polypeptide sequences may be examined using the following unix command line parameters:
b12seq -i peptideseq1 -j peptideseq2 -F F -p blastp

Variant polypeptide sequences preferably exhibit an E value of less than 1 x 10⁻¹⁰, more preferably less than 1 x 10⁻²⁰, less than 1 x 10⁻³⁰, less than 1 x 10⁻⁴⁰, less than 1 x 10⁻⁵⁰, less than 1 x 10⁻⁶⁰, less than 1 x 10⁻⁷⁰, less than 1 x 10⁻⁸⁰, less than 1 x 10⁻⁹⁰, less than 1 x 10⁻¹⁰⁰, less than 1 x 10⁻¹¹⁰, less than 1 x 10 ⁻¹²⁰, or less than 1 x 10 ⁻¹²³ when compared with any one of the specifically identified sequences.

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. For small E values, much less than one, this is approximately the probability of such a random match.

Conservative substitutions of one or several amino acids of a described polypeptide sequence without significantly altering its biological activity are also included in the invention. A skilled artisan will be aware of methods for making phenotypically silent amino acid substitutions (see, e.g., Bowie et al, 1990, Science 247, 1306).

A polypeptide variant for use in the present invention also encompasses that which is produced from the nucleic acid encoding a polypeptide, but differs from the wild type polypeptide in that it is processed differently such that it has an altered amino acid sequence. For example a variant may be produced by an alternative splicing pattern of the primary RNA transcript to that which produces a wild type polypeptide.

Multiple sequence alignments of a group of related sequences can be carried out with CLUSTALW (Thompson, J.D., Higgins, D.G. and Gibson, T.J. (1994) CLUSTALW: improving the sensitivity of progressive multiple sequence alignment through sequence weighting, positions-specific gap penalties and weight matrix choice. Nucleic Acids Research, 22:4673- 4680, http://www-igbmc.u-strasbg.frBioInfo/ClustalW/Top.html) or T-COFFEE (Notredame et al, J. Mol. Biol, 302:205-217, 2000) or PILEUP, which uses progressive, pairwise alignments. (Feng and Doolittle, J. Mol Evol, 25:351, 1987).

Pattern recognition software applications are available for finding motifs or signature sequences. For example, MEME (Multiple Em for Motif Elicitation) finds motifs and signature sequences in a set of sequences, and MAST (Motif Alignment and Search Tool) uses these motifs to identify similar or the same motifs in query sequences. The MAST results are provided as a series of alignments with appropriate statistical data and a visual overview of the motifs found. MEME and MAST were developed at the University of California, San Diego.

PROSITE (Bairoch and Bucher, Nucl. Acids Res., 22:3583, 1994; Hofmann et al., Nucl. Acids Res., 27:215, 1999) is a method of identifying the functions of uncharacterized proteins translated from genomic or cDNA sequences. The PROSITE database (www.expasy.org/prosite) contains biologically significant patterns and profiles and is designed so that it can be used with appropriate computational tools to assign a new sequence to a known family of proteins or to determine which known domain(s) are present in the sequence (Falquet et al., Nucl. Acids Res., 30:235, 2002). Prosearch is a tool that enables a user to search a number of databases including SWISS-PROT, SWISS-2DPAGE, SWISS-3DIMAGE, and ENZYME, as well as other cross-referenced databases such as EMBL, GenBank, OMIM, Medline databases, etc., with a given sequence pattern or signature.

In addition to the computer/database methods described above, polypeptide variants may be identified by physical methods, for example by screening expression libraries using antibodies raised against FNR polypeptides used in the invention (Sambrook et al, Molecular Cloning: A Laboratory Manual, 2nd Ed. Cold Spring Harbor Press, 1987) or by identifying polypeptides from natural sources with the aid of such antibodies.

### Therapeutic/Prophylactic Administration and Compositions

The invention provides methods of treatment (and prophylaxis) by administration to a subject of an effective amount of a compound of the invention. In a preferred aspect, the Therapeutic is substantially purified. The subject is preferably an animal, including but not limited to animals such as cows, pigs, horses, chickens, cats, dogs, etc., and is preferably a mammal, and most preferably human. In a specific embodiment, a non-human mammal is the subject.

Various delivery systems are known and can be used to administer a Therapeutic of the invention, e.g., encapsulation in liposomes (Langer, Science 249:1527-1533 (1990); Treat et al., in Liposomes in the Therapy of Infectious Disease and Cancer, Lopez-Berestein and Fidler (eds.), Liss, New York, pp. 353-365 (1989)), microparticles, microcapsules, recombinant cells capable of expressing the Therapeutic, receptor-mediated endocytosis (see, e.g., Wu and Wu, 1987, J. Biol. Chem. 262:4429-4432), construction of a Therapeutic nucleic acid as part of a retroviral or other vector, etc. Methods of introduction include intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (e.g., oral mucosa, rectal and intestinal mucosa, etc.) and may be administered together with other biologically active agents.

The present invention also provides pharmaceutical compositions. Such compositions comprise a therapeutically effective amount of a Therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognised pharmacopeia for use in animals, and more particularly in humans. The term "carrier" refers to a diluent, adjuvant, excipient, or vehicle with which the Therapeutic is administered. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Examples of suitable pharmaceutical carriers are described in "Remington's Pharmaceutical Sciences" by E.W. Martin. Such compositions will contain a therapeutically effective amount of the Therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilising agent and a local anesthetic such as lignocaine to ease pain at the site of the injection.

The amount of the Therapeutic of the invention which will be effective in the treatment of a particular disorder or condition will depend on the nature of the disorder or condition, and can be determined by standard clinical techniques. In addition, *in vitro* assays may optionally be employed to help identify optimal dosage ranges. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient I the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention.

### Screening for FNR Agonists and Antagonists

FNR nucleic acids, proteins, and derivatives may be used in screening assays to detect molecules that specifically bind to FNR nucleic acids, proteins, or derivatives and thus have potential use as agonists or antagonists of FNR, in particular molecules that inhibit FNR dimerisation or nucleic acid binding. In a preferred embodiment, such assays are performed to screen for molecules with potential utility as lead compounds for drug development. The invention thus provides assays to detect molecules that specifically bind to FNR nucleic acids, proteins, or derivatives or bind to or interfere with the formation of FNR dimers. For example, recombinant cells expressing FNR nucleic acids can be used to recombinantly produce FNR proteins in these assays, to screen for molecules that bind to a FNR protein, and recombinant cells expressing FNR and FNR target nucleic acids can be used to screen for molecules that bind to or inhibit formation of an FNR-DNA complex. Molecules are contacted with the FNR protein (or fragment thereof) under conditions conducive to binding, and then molecules that specifically bind to the lats protein or bind to or interfere with the formation of FNR dimers or FNR-DNA complexes are identified.

By way of example, diversity libraries, such as random or combinatorial peptide or nonpeptide libraries can be screened for molecules that specifically bind to FNR. Many libraries are known in the art that can be used, e.g., chemically synthesized libraries, recombinant (e.g., phage display libraries), and *in vitro* translation-based libraries.

Examples of chemically synthesized libraries are described in Fodor et al., 1991, Science 251:767-773; Houghten et al., 1991, Nature 354:84-86; Lam et al., 1991, Nature 354:82-84; Medynski, 1994, Bio/Technology 12:709-710; Gallop et al., 1994, J.Medicinal Chemistry 37(9):1233-1251; Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; Salmon et al., 1993, Proc. Natl. Acad. Sci. USA 90:11708-11712; PCT Publication No. WO 93/20242; and Bremner and Lerner, 1992, Proc. Natl. Acad. Sci. USA 89:5381-5383.

Examples of phage display libraries are described in Scot and Smith, 1990, Science 249:386-390; Devlin et al., 1990, Science, 249:404-406; Christian, R.B., et al., 1992, J. Mol. Biol. 227:711-718; Lenstra, 1992, J. Immunol. Meth. 152:149-157; Kay et al., 1993, Gene 128:59-65; and PCT Publication No. WO 94/18318 dated August 18, 1994.

*In vitro* translation-based libraries include but are not limited to those described in PCT Publication No. WO 91/05058 dated April 18, 1991; and Mattheakis et al., 1994, Proc. Natl. Acad. Sci. USA 91:9022-9026.

By way of examples of nonpeptide libraries, a benzodiazepine library (see e.g., Bunin et al., 1994, Proc. Natl. Acad. Sci. USA 91:4708-4712) can be adapted for use. Peptoid libraries (Simon et al., 1992, Proc. Natl. Acad. Sci. USA 89:9367-9371) can also be used. Another example of a library that can be used, in which the amide functionalities in peptides have been permethylated to generate a chemically transformed combinatorial library, is described by Ostresh et al. (1994, Proc. Natl. Acad. Sci. USA 91:11138-11142).

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992; BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al., Rebar and Pabo, 1993, Science 263:671-673; and PCT Publication No. WO 94/18318.

In a specific embodiment, screening can be carried out by contacting the library members with an FNR protein (or nucleic acid or derivative) immobilized on a solid phase and harvesting those library members that bind to the protein (or nucleic acid or derivative). Examples of such screening methods, termed "panning" techniques are described by way of example in Parmley and Smith, 1988, Gene 73:305-318; Fowlkes et al., 1992, BioTechniques 13:422-427; PCT Publication No. WO 94/18318; and in references cited herein above.

In another embodiment, the two-hybrid system for selecting interacting proteins in yeast (Fields and Song, 1989, Nature 340:245-246; Chien et al., 1991, Proc. Natl. Acad. Sci. USA 88:9578-9582) can be used to identify molecules that specifically bind to an FNR protein or derivative or that interfere with the formation of FNR dimers or FNR-DNA complexes.

Fnr can repress or activate gene expression under anaerobic conditions. Transcriptional activity of FNR can be monitored by several methods including Northern analysis and RT-PCR. Additionally genetic fusions can be constructed between promoter sequences (1) and reporter genes. The advantage of user reporter genes is that the level of products can be measured simply. This can be performed either enzymatically (for instance using fusions with LacZ and XylE (2)), or using fluorescence-based methods (such as green fluorescent protein, GFP, its variants or other fluorescent markers (3)). Reporter fusions can be constructed on multi-host plasmids and maintained in *Shigella* and E. *coli.* By selecting appropriate promoter sequences, the activity of Fnr in a bacterium can be simply assayed.

However, a significant obstacle to using bacteria harbouring reporter strains for performing high-throughput screening is the need to maintain the system under anaerobic conditions which are necessary for Fnr activity. This problem can be overcome by using a constitutively active version of Fnr (for example that disclosed in reference 4), which is not dependent on the lack of oxygen to be able to bind DNA and stimulate gene transcription.

Therefore screening can be established by introducing a plasmid harbouring a reporter fused to an Fnr dependent promoter into a bacterium which expresses a constitutively active form of Fnr. Under these circumstances, the promoter should be active regardless of the ambient oxygen concentration, and the function of product of the reporter should be detected.

This screening could be most safely and efficiently carried out using a non-pathogenic bacterium such as a disabled E. *coli* host. This would still be an effective screen given the 100% amino acid identity between Fnr in *Shigella* and E. *coli.*

Compounds that inhibit Fnr function would be identified by their ability to prevent reporter activity. Clearly a compound may prevent reporter activity directly and not by inhibiting Fnr. This possibility could be excluded by performing the assay in duplicate with two independent reporters. Furthermore, the activity of the promoter could be monitored in the presence of selected hit compounds in the presence and absence of oxygen in bacteria which contain a normal version of Fnr.

### EXAMPLES

### Example 1 - Identification of a highly attenuated Shigella strain

During a signature tagged mutant screen of 2,896 *S. flexneri* Tn5 mutant strains, an *fnr* (fumarate nitrate reduction) mutant was identified that was significantly attenuated in rabbit ileal loops, the most validated model of intestinal shigellosis.

New Zealand White rabbits weighing 2.5-3 kg (Charles River Breeding Laboratories, Wilmington, MA) were used for experimental infections. From each animal, up to 12 intestinal ligated loops, each 5 cm in length, were prepared as described previously (5). Bacteria were injected in 0.5 ml of saline into each loop. For evaluation of the competitive index (CI) of a mutant, each loop received a total dose of 105 CFU, and colonisation-defective mutants were identified by their failure to be recovered 16 h later. Animals were sacrificed at this time, the lumenal fluid aspirated, and *S*. *flexneri* recovered. The C.I. was calculated as the proportion of mutant to wild-type bacteria recovered from animals, divided by the proportion of mutant to wild-type in the inoculum, and results are expressed as the mean of at least 4 loops from two independent animals for each strain tested. For evaluation of virulence, loops were dissected, a segment was opened longitudinally, and fixed in 4 % buffered formalin. Sections (5 µm) were obtained, and following haematoxylin-eosin-safranin (HES) and Giemsa staining, histopathological observation and analysis was performed. For each mutant and control strains, at least four samples originating from four different rabbits were examined.

Histopathological changes following challenge with of rabbit ileal loops with the *fnr* mutant, wild-type (M90T), the complemented strain (*fnr* pBM2) and a non-invasive strain (*mxiD*) are shown in Figure 1. The *fnr* mutant had a competitive index (CI) of 0.025, while the CI of the complemented strain was 0.67. The attenuation of the *fnr* mutant was confirmed by the lack of bacterial invasion and inflammatory response in rabbit ileal loops challenged with the *fnr* mutant.

### Example 2 - Mediation of Shigella virulence by fnr

To investigate the influence of available oxygen on the behaviour of *S. flexneri,* the capacity of the bacterium to invade human epithelial cells, an essential step in virulence, was examined under aerobic and anaerobic conditions.

Invasion assays were performed with HeLa epithelial cells seeded onto glass coverslips in 12-well tissue culture plates (Life Technologies), and grown until they were semi-confluent. *S. flexneri* strains were diluted from overnight liquid cultures and grown at 37°C until they reached an optical density of 0.2 at *A*₆₀₀. Cells were infected with wild-type *S*. *flexneri* (M90T), *the fnr* mutant (*fnr⁻*) or the complemented strain (*fnr* pBM2) at an MOI of 100. After 10 min (for initial adhesion) and one hour (for invasion) at 37°C, cells were washed three times with PBS and incubated for a further hour with gentamicin (100 µg/ml). Viable bacteria were counted by plating serial dilutions. Results were averaged from three independent experiments, each performed with triplicate wells. The Invasion Index was calculated as the ratio of internalised to adherent bacteria.

As shown in Figure 2A, the invasion of *S. flexneri* was increased by an order of magnitude in an anaerobic environment. This effect was not influenced by the host cell response to hypoxia. No change in bacterial invasion was observed either under aerobic conditions in cells with or without Von Hippel-Lindau factor (necessary for the degradation of the transcription factor Hypoxia inducible factor, HIFα), or under anaerobic conditions following inhibition of HIF with L-mimosine (not shown).

Western analysis of proteins from strains probed with α-FNR antibodies (Prof. J. Green) was used to confirm that wild-type and complemented *S. flexneri* express FNR, in contrast to the mutant, as shown as "α-FNR" in Figure 2A.

Immunofluorescence analysis was also used to examine bacterial invasion under anaerobic conditions. For immunofluorescence analysis, bacteria and cells were fixed (PFA 3%). Bacteria were stained using a rabbit anti-*Shigella* LPS primary antibody (P. Sansonetti, Institut Pasteur) and an anti-rabbit Cy2-conjugated secondary antibody. Cells were stained using a conjugated Rhodamine-phalloidin antibody to detect actin. Image acquisition was then performed using laser-scanning confocal microscopy, and the results shown in Figure 2B.

Analysis of the *fnr* mutant and complemented strain demonstrated that *fnr* is essential for the enhanced hyper-invasive state of the bacterium grown in anaerobic conditions.

### Example 3 - Pathology of Shigella infection in rabbit ileal loops

The pathology in rabbit ileal loops was also compared 18 hrs after challenge with wild-type bacteria grown under aerobic or anaerobic conditions.

Figure 3 shows the histological analysis of infected rabbit ileal loops with the wild-type strain (M90T) grown aerobically or anaerobically (indicated with a + or -, respectively). Bacteria were detected with an anti-LPS antibody, and sections counterstained with Evans blue. Lumenal crypts are shown by asterisks.

Anaerobically-grown bacteria were found deep within the intestinal crypts, and this was not observed following inoculation with aerobically-grown *Shigella.* Furthermore, there was increased tissue destruction seen in loops infected with anaerobically-grown bacteria. Together these results demonstrate that anaerobic growth promotes a hyper-invasive phenotype of *Shigella* and increased virulence *in vivo.*

### Example 4 - Mediation of the Type Three Secertory System by FNR

In this example, the profile of effectors secreted by the Type Three Secertory System (TTSS) was examined under different conditions.

An overnight culture was sub-cultured into 1 L TCS media and grown until OD 0.2, to ensure a full aerobiasis of the culture. Bacteria were harvested and washed in 10 mL PBS before being resuspended in 100 µL PBS. Secretion was induced, or not, by adding 1% Congo Red and further incubating 20 min at 37°C. Supernatants and whole cell extracts were collected and separated by SDS-PAGE. Proteins were detected by Coomassie blue staining. Western blotting was performed with rabbit anti-IpaB, anti-IpaC, anti-IpaD and anti-RecA antibodies. Binding of antibodies was detected using an ECL kit (Amersham Biosciences).

Strikingly, the induction of Invasion Protein Antigen (Ipa) secretion upon exposure to Congo red (CR, a widely used but artificial inducer of TTSS secretion) was not seen in anoxic conditions (boxed, Figure 4A). This lack of secretion was not seen in the *fnr* mutant, and was not accompanied by changes in intracellular levels of Ipas (Figure 4B) or in the amount or type of lipopolysaccharide (not shown). These results demonstrate that anaerobiasis inhibits the secretion of Ipa effectors by *Shigella,* and this is mediated by FNR.

### Example 5 - Priming of Shigella invasiveness

The results in Example 4 demonstrate that anaerobiasis influences secretion through the TTSS. In this experiment, the appearance of *Shigella* was examined by scanning EM. There were significantly more secretons detected on the surface of cells grown under anaerobic conditions compared with those grown with full oxygenation (Figure 5), consistent with increased secretion of TTSS needle components or an alteration in the needle length in the lack of oxygen.

Therefore in an anaerobic environment, such as the lumen of the GI tract, *Shigella* is primed for invasion, by reducing secretion of Ipa effectors while increasing the number of accessible needles to engage host cells. This 'primed' state is reflected by the hyper-invasiveness of bacteria in anaerobic conditions (Figure 1).

### Example 6 - Binding of FNR upsteam of Shigella virulence genes

The results of the previous examples suggest that FNR modulates transcription of genes on the *Shigella* virulence plasmid, particularly those control substrate secretion by the TTSS.

*In E. coli,* the DNA consensus binding sequence of FNR is TTGAT(N₄)ATCAA. On the assumption that that any variability can be observed in this regulatory sequence, the following sequences in the virulence plasmid: TTGAT(N₄)AT were screened. Several potential FNR-binding consensus sequences upstream of virulence genes in the *mxi*/*spi* locus were identified, and DNA gel shift assays were performed to demonstrate that active FNR binds to the promoter regions of three of four genes tested to date (Figure 6), including *spa33* and *mxiM.* While the precise function of Spa33 is unknown, over-expression of this protein blocks Ipa secretion. *MxiM* is a lipoprotein required for Ipa translocation. Additionally there are two FNR consensus sequences upstream of *Spa32* which is an ATPase that controls needle length and the selection of substrates targeted for secretion.

Purified FNR D¹⁵⁴A binds to DNA fragments of regions upstream of *spa33* and *mxiM.* Electrophoretic Mobility Shift Assays (EMSA) using purified FNR D¹⁵⁴A protein was carried out as detailed previously (6). Purified *mxiM* and *spa33* promoter fragments were end labelled with [γ-³²P]-ATP, and ≈ 0.5 ng of each fragment was incubated with varying amounts of each protein. The reaction buffer contained 10 mM potassium phosphate (pH 7.5), 100 mM potassium glutamate, 1 mM EDTA, 50 µM DTT, 5% glycerol and 25 µg ml⁻¹ herring sperm DNA. The final reaction volume was 10 µl. After incubation at 37°C for 20 min, samples were run in 0.25× standard TBE buffer on a 6% polyacrylamide gel (12 V cm⁻¹), containing 2% glycerol, and analysed by either autoradiography or using a Bio-Rad Molecular Imager FX and Quantity One software (Bio-Rad). The results of the EMSA assays are shown in Figure 6.

### Example 7 - Presence of FNR in many bacterial pathogens

FNR belongs to a large family of regulators in bacteria called the Crp/Fnr family. The Crp-Fnr proteins are similar in size and are composed of around with 250 amino acids. They contain C-terminus helix-turn-helix (HTH) domain (which mediates binding to DNA), which consists of two α-helices linked by a turn, which fits into the major groove of DNA. Furthermore, Crp-Fnr regulators have a large nucleotide-binding domain at the N-terminus. These two domains define members of the family (7). Members of the Crp/Fnr family are found throughout bacteria including Gram negative pathogens and in gram positives. Proteins that are closely related to Fnr are found in *E. coli, Shigella, Salmonella,* and multi-resistant organisms including *Pseudomonas* and *Klebsiella.* It was originally thought that close homologues of FNR were restricted to Gram negatives, but the identification of FNRb in *Bacillus* demonstrated that this view was incorrect (8). Related proteins are also found in *Clostridium* species, including *C*. *difficile* which also inhabits the gastrointestinal tract (Table 1). Furthermore there are FNR homologues in oral pathogens *Capnocytophaga ochracea, Capnocytophaga sputigena, Haemophilus aphrophilus, and Actinobacillus actinomycetemcomitans* (9). FNR sequences from Gram negative bacteria (*Shigella flexneri* (SFV_1825), *Salmonella typhimurium LT2 SGSC1412* (STM1660), *Vibrio cholerae* (VC_1434), *Yersinia pestis 092* (YPO2300) and *Neisseria meningitidis* (NMB_0380)) and Gram positive (*Bacillus subtilis* (Bsu3729)) were compared by sequence alignment (using Clustalw software) and shown in Table 1.

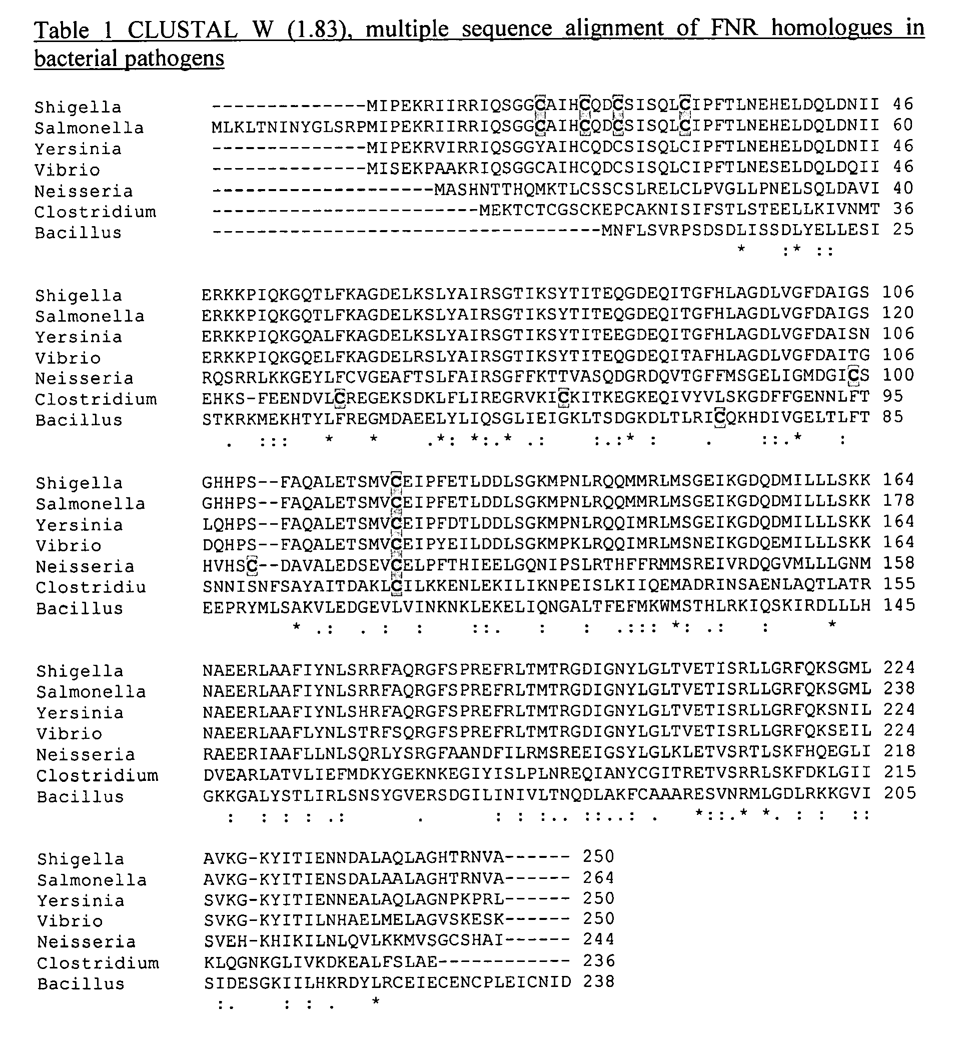

### The following references are incorporated herein by reference

1) Tyson KL, et al., Definition of nitrite and nitrate response elements at the anaerobically inducible Escherichia coli nirB promoter: interactions between FNR and NarL. Mol Microbiol. 1993 7: 151-7.
2) Miller WG, et al., An improved GFP cloning cassette designed for prokaryotic transcriptional fusions. Gene. 1997 191: 149-53.
3) Prigent-Combaret C, et al., Monitoring gene expression in biofilms. Methods Enzymol. 1999 310: 56-79.
4) Ziegelhoffer EC, et al., In vitro analysis of a constitutively active mutant form of the Escherichia coli global transcription factor FNR. J Mol Biol. 1995 245: 351-61.
5) Sansonetti P.J. et al., Infection of rabbit Peyer's patches by Shigella flexneri: effect of adhesive or invasive bacterial phenotypes on follicle-associated epithelium Infect. Immun. 1996 64: 2752-2764.
6) Bates, D. M., et al., (2000). Substitution of Leucine 28 with Histidine in the Escherichia coli Transcription Factor FNR Results in Increased Stability of the [4Fe-4S]2+ Cluster to Oxygen. J. Biol. Chem. 275: 6234-6240.
7) Korner H, et al. Phylogeny of the bacterial superfamily of Crp-Fnr transcription regulators: exploiting the metabolic spectrum by controlling alternative gene programs. FEMS Microbiol Rev. 2003 27:559-92.
8) Cruz Ramos H, et al. Anaerobic transcription activation in Bacillus subtilis: identification of distinct FNR-dependent and -independent regulatory mechanisms. EMBO J. 1995 14:5984-94.
9) Hattori T, et al. Novel FNR homologues identified in four representative oral facultative anaerobes: Capnocytophaga ochracea, Capnocytophaga sputigena, Haemophilus aphrophilus, and Actinobacillus actinomycetemcomitans. FEMS Microbiol Lett. 1996 137:213-20.

## Claims

1. A compound capable of inhibiting FNR transcription factor activity in a bacterial pathogen.

2. A compound as claimed in claim 1 which is an antibody or epitope binding fragment or derivative thereof which inhibits FNR transcription factor activity by inhibiting dimerization of active FNR monomers.

3. A compound as claimed in claim 2 which is an antibody or epitope binding fragment or derivative thereof which inhibits FNR transcription factor activity by inhibiting conformational changes in response to low oxygen tension.

4. A compound as claimed in claim 2 which is an antibody or epitope binding fragment or derivative thereof which inhibits FNR transcription factor activity by disrupting binding of FNR dimers to target DNA sequences.

5. A compound as claimed in claim 1 which is an inhibitory nucleic acid molecule capable of hybridizing to the following consensus sequence under conditions found in *vivo:*
TTGAT(N₄)ATCAA where N is any residue, or an antisense molecule capable of reducing FNR expression.

6. A compound as claimed in one of claims 1 to 5 which inhibits transcription of genes of the TTSS.

7. A compound as claimed in one of claims 1 to 6 which inhibits cellular invasion of genes of the TTSS.

8. A compound as claimed in one of claims 1 to 7 wherein said bacterial pathogen is a human pathogen.

9. A compound as claimed in one of claims 1 to 7 wherein said bacterial pathogen is a pathogen of non-human animals.

10. A compound as claimed in one of claims 1 to 7 wherein said bacterial pathogen is a pathogen of plants.

11. A compound as claimed in claim 8 wherein said pathogen is a gram negative pathogen selected from *Pseudomonas, Klebsiella, Salmonella, E. Coli,* and *Shigella.*

12. A compound as claimed in claim 11 wherein said pathogen is *Shigella.*

13. A compound as claimed in claim 8 wherein said pathogen is a gram positive pathogen selected from *Clostridium* and *Bacillus.*

14. A method of treating or preventing a pathogenic infection caused by a pathogenic bacteria as defined in any of said claims 8 to 13 by administering to a human, animal or plant a compound as defined in one of claims 1 to 13.

15. A compound as defined in any claims 1 to 9, or 11 to 13 for use in the treatment of a disease caused by a bacterial pathogen as defined in any one of claims 8 to 13.

16. A compound as claimed in claim 15 for use in treating bacillary dysentery caused by *Shigella.*

17. A method of screening for a compound capable of inhibiting FNR transcription factor activity comprising the following steps:
i) contacting a candidate compound with a bacteria expressing FNR and comprising a genetic construct comprising an FNR-dependent promoter operably linked to a reporter gene.
ii) detecting inhibition of expression of the reporter gene.

18. A method as claimed in claim 17 carried out under anaerobic conditions.

19. A method as claimed in claim 18 carried out under aerobic conditions, wherein the bacteria comprises a constitutively active version of FNR which is not dependent on lack of oxygen to be able to find FNR and stimulate gene transcription.

20. A method as claimed in claim 17, claim 18 or claim 19 wherein said bacteria is a disabled *E.coli* bacteria.

21. A method as claimed in claim 20 wherein said disabled *E.coli* bacteria is engineered to express a *Shigella* FNR protein.

22. A compound identified according to a method as claimed in any of claims 17 to 21.
